# EUROPÄISCHE PATENTSCHRIFT

(11) **EP 4 021 354 B1**
(45) Veröffentlichungstag und Bekanntmachung des Hinweises auf die Patenterteilung: **21.06.2023**
(21) Anmeldenummer: 20761557.6
(22) Anmeldetag: 24.08.2020
(51) Int. Cl.: A61F 5/01, A61F 5/30

(54) **PELOTTE UMFASSEND EIN DRUCKELEMENT**
PAD COMPRISING A PRESSURE ELEMENT
COUSSINET COMPRENANT UN ÉLÉMENT DE PRESSION

(30) Priorität: 26.08.2019 DE 102019212740
(43) Veröffentlichungstag der Anmeldung: 06.07.2022
(73) Patentinhaber: Bauerfeind AG, 07937 Zeulenroda-Triebes (DE)
(72) Erfinder: BAUERFEIND, Hans B., 07937 Zeulenroda (DE)
(74) Vertreter: Wohlfahrt, Jan Günther
(86) Internationale Anmeldenummer: PCT/EP2020/073613
(87) Internationale Veröffentlichungsnummer: WO 2021/037789

(56) Entgegenhaltungen:
- DE-A1- 4 103 383
- DE-A1-102012 021 696
- DE-A1-102017 108 840
- DE-C2- 4 103 383

## Beschreibung

Die Erfindung betrifft Pelotten, insbesondere für orthopädische Hilfsmittel, umfassend ein Pelottendruckelement. Die Erfindung betrifft auch die Verwendung der erfindungsgemäßen Pelotten in Orthesen oder Bandagen sowie Orthesen oder Bandagen, die erfindungsgemäße Pelotten aufweisen.

Pelotten sind in verschiedenen Ausführungsformen und für verschiedene prophylaktische und therapeutische Anwendungen bekannt. Verschiedene Pelotten sind beispielsweise aus der DE 27 22 563 C2, der EP 0 598 291 A1 und der EP 0 600 218 A2 bekannt.

Auch sind Pelotten aus zwei Materialien oder Komponenten aus der DE 297 01001 U1 bekannt. Die EP 0 496 071 A1 beschreibt eine Druckpelotte aus einem weicheren Material, in dem mindestens ein Friktionskern aus einem harten oder inkompressiblen Material angeordnet ist. Dieser Friktionskern soll einer Friktionsmassage, also einer Massage durch Bewegungsreibung von Schmerzpunkten dienen, die durch die Bewegung des Kerns gegenüber dem Weichgewebe des Pelottenträgers erzeugt wird.

Die DE 10 2012 021 696 A1 betrifft ein Element für ein zumindest teilweise viscoelastisches orthopädisches Medizinprodukt oder für ein zumindest teilweise viscoelastisches orthopädisches Hilfsmittel, wobei das Element mindestens eine erste schichtförmige Komponente und eine zweite schichtförmige Komponente enthält, wobei die erste Komponente härter ist als die zweite Komponente, wobei eine Fläche der ersten Komponente mit einer Fläche der zweiten Komponente verbunden ist und wobei das Element über seine Fläche mindestens zwei Zonen unterschiedlicher Härte aufweist, dadurch gekennzeichnet, dass die mit der zweiten Komponente verbundene Fläche der ersten Komponente eine Vielzahl von Erhebungen aufweist, wobei über die Höhe, Breite und/oder die Verteilung der Erhebungen in einer Zone des Elements die Härte dieser Zonen bestimmt wird.

Die Pelotten aus dem Stand der Technik können flächig einen Druck auf das anliegende Gewebe ausüben oder anfallenden Druck auf die Fläche verteilen und/oder abschirmen, jedoch ist mit diesen Pelotten die Ausübung eines spezifischen Drucks, beispielsweise eines stärkeren oder schwächeren Drucks auf spezifische Punkte nicht oder nur unzureichend möglich.

Auch erfolgt der Druck bei Pelotten aus dem Stand der Technik senkrecht auf die Haut. Ein seitlicher Druck, der insbesondere zu einer Bewegung der Druckelemente führt und somit einen Massageeffekt über den einfachen Druck hinaus herbeiführt, ist so nicht möglich.

Das der vorliegenden Erfindung zugrundeliegende technische Problem ist daher die Bereitstellung verbesserter Pelotten, insbesondere solcher Pelotten, die neben einem flächigen Druck einen spezifischen punktgenauen Druck ausüben können. Insbesondere ist das der vorliegenden Erfindung zugrundeliegende technische Problem die Bereitstellung einer Pelotte, die zu einem verbesserten Massageeffekt führt, insbesondere zu einem Massageeffekt durch Bewegung des Druckkörpers.

Das der Erfindung zugrunde liegende technische Problem wird gelöst durch die Bereitstellung einer Pelotte für orthopädische Hilfsmittel, umfassend einen Pelottengrundkörper aus einem ersten Material und mindestens einem Pelottendruckelement aus einem zweiten Material, wobei das Pelottendruckelement zumindest teilweise in den Pelottengrundkörper eingebettet ist und wobei das erste Material des Pelottengrundkörpers weicher ist als das zweite Material des Pelottendruckelements, wobei das Pelottendruckelement mindestens zwei zapfenförmige Erhebungen und ein Grundelement aufweist, wobei die zapfenförmigen Erhebungen auf dem Grundelement angeordnet sind, wobei die zapfenförmigen Erhebungen über das Grundelement viskoelastisch beweglich sind, wobei das Grundelement mindestens ein Loch aufweist und wobei die mindestens zwei zapfenförmige Erhebungen auf dem Grundelement am Rand des mindestens einen Lochs positioniert sind, dadurch gekennzeichnet dass die mindestens zwei zapfenförmigen Erhebungen über einen das Loch überspannenden Steg miteinander verbunden sind und sich mindestens eine weitere zapfenförmige Erhebung auf dem Steg befindet.

Die Erfindung zeichnet sich also vor allem dadurch aus, dass die mindestens zwei zapfenförmigen Erhebungen über einem Grundelement miteinander verbunden sind und über dieses viskoelastisch beweglich sind, wenn sie zumindest teilweise in den Pelottengrundkörper eingebettet sind.

Die viskoelastische Beweglichkeit der zapfenförmigen Erhebungen, insbesondere zueinander, kann bevorzugt durch die biegsame Ausgestaltung des Grundelements erreicht werden. Daher ist das Grundelement bevorzugt biegsam.

Der erfindungsgemäße Aufbau der Pelotte mit einem neuartigen Pelottendruckelement aus über ein Grundelement verbundenen zapfenförmigen Erhebungen, die zumindest teilweise im weicheren Pelottengrundkörper eingebettet sind und die über das Grundelement viskoelastisch beweglich sind, führt in vorteilhafter Weise zu einem verbesserten Massageeffekt, insbesondere zu einer Friktionsmassage, bei der auf die Haut nicht nur ein Druck ausgeübt wird, sondern auch eine seitliche Bewegung stattfindet. Es zeigte sich, dass bei der erfindungsgemäßen Ausgestaltung die sich über das Grundelement miteinander verbundenen Zapfen insbesondere an ihren vom Grundelement entfernt liegenden Enden zueinander bewegen oder voneinander weg bewegen, wenn die Pelotte senkrecht auf die Haut des Patienten gedrückt wird. Das auseinander Bewegen beziehungsweise zueinander Bewegen der zapfenförmigen Erhebungen stellt in vorteilhafter Weise eine seitliche Bewegung dar, die durch den senkrechten Druck der Pelotte auf die Haut verursacht wird und die zu einer verbesserten Massage führt. Dabei stellte sich in vorteilhafter Weise heraus, dass diese seitliche Bewegung chaotisch erfolgt, was den Massageeffekt verstärkt.

Die erfindungsgemäß über das Grundelement verbundenen, also besonders bevorzugt auch speichengelagerten zapfenförmigen Erhebungen federn im Vergleich zu Erhebungen aus dem Stand der Technik wesentlich freier und beweglicher, und somit insbesondere auch chaotischer. Dieser Effekt ist vorteilhaft, da sich die direkt unter der Hautoberfläche liegenden Mechano-Rezeptoren auf die immer wieder etwas veränderte Federbewegung der zapfenförmigen Erhebungen schwerer einstellen können. Dies führt zu einer verlängerten Wirkung der Massage.

Da bei einer manuellen Therapie "chaotisch" massiert wird, da kein Griff des Masseurs zu 100% dem anderen gleicht, ist die durch die erfindungsgemäße Pelotte entstehende Massagewirkung der einer manuellen Massage ähnlicher als die Massagewirkung durch herkömmliche Pelotten. Die Verbindung von zapfenförmigen Erhebungen, beispielsweise noppenartigen Erhebungen, mit daran verbundenen Grundelementen, wie Stegen, die bevorzugt auch speichenartig Löcher in dem Grundflächenkörper überspannen, führt in vorteilhafter Weise zu beweglichen Erhebungen, die ein intermittierendes Massageprinzip ermöglichen. Während unbewegliche Erhebungen auf einer Pelotte nur eine Druckwirkung haben, die auf die Hautsensoren wirkt, die jedoch nach relativ kurzer Zeit an den Druck gewöhnt sind und keine wirksamen Signale mehr aussenden, führen die erfindungsgemäßen beweglichen zapfenförmigen Erhebungen zu einem Wechseldruck, wie ihn zum Beispiel auch ein Therapeut bei einer Massage ausübt, sodass die Druckwirkung auf Lymph- und Blutgefäße bestehen bleibt. Dies kann insbesondere beispielsweise bei einer Kniepelotte vorteilhaft sein, wenn dort ein entsprechender Massageeffekt bei der Meniskusbasis und den Hoffaschen Fettkörpern ansetzt. Es zeigte sich darüber hinaus überraschenderweise, dass bei einem Einsatz des Pelottendruckelements ohne umhüllende Komponente die äußeren zapfenförmigen Elemente meist nach innen knicken, wenn auf ein inneres auf einem Steg liegendes zapfenförmiges Element Druck ausgeübt wird, dagegen aber die außenliegenden zapfenförmigen Elemente bei einem von einem Pelottenkörper umhüllten erfindungsgemäßen Pelottendruckelement meist nach außen knicken, wenn auf ein inneres auf einem Steg liegendes zapfenförmiges Element Druck ausgeübt wird. Beide Effekte sind vorteilhaft, da sie zu einer Bewegungsmassage führen, die der manuellen Massage ähneln.

Während ein auseinander Bewegen der zapfenförmigen Elemente zu einer seitwärts gerichteten Zugbewegung führt, führt das zueinander Bewegen zu einer seitwärts gerichteten Druckbewegung. Es zeigte sich überraschender Weise, dass diese Bewegung sich auch dann deutlich auf die Haut überträgt, wenn die zapfenförmigen Elemente in den weicheren Pelottengrundkörper zumindest teilweise eingebettet, beispielsweise eingegossen, sind.

Unter einer Pelotte wird im Zusammenhang mit der vorliegenden Erfindung insbesondere eine Druckpelotte verstanden, deren Form insbesondere durch den Pelottengrundkörper gebildet wird. Der Pelottengrundkörper kann jegliche geeignete Form aufweisen. Herkömmliche Pelotten weisen typischerweise ebene Grundflächen auf. Aus einer solchen Grundfläche können dann Vorsprünge herausragen. Eine erfindungsgemäße Pelotte weist insbesondere eine erste Grundfläche oder Grundoberfläche auf, die bei Verwendung der Pelotte dem Benutzer zugewandt ist. Bevorzugt ist eine Pelotte im Wesentlichen flach ausgestaltet. Eine solche Pelotte weist dann bevorzugt eine zweite Grundfläche oder Grundoberfläche auf, die dem Benutzer bei der Verwendung der Pelotte abgewandt ist. Die zapfenförmigen Erhebungen zeigen vom Pelottengrundkörper in Richtung des Benutzers.

Die typische Verwendung einer Pelotte ist dem Fachmann bekannt. Pelotten, auch die erfindungsgemäßen Pelotten, werden insbesondere dazu verwendet, auf bestimmte Körperpartien, beispielsweise im Bereich des Rückens oder im Bereich der Kniescheibe einen Druck auszuüben. Dabei werden die Pelotten positioniert und über weitere Vorrichtungen, insbesondere Bandagen oder Orthesen auf den entsprechenden Körperbereich gedrückt.

In einer bevorzugten Ausführungsform ist zumindest ein Teilbereich der mindestens zwei zapfenförmigen Erhebungen in den Pelottengrundkörper eingebettet. Bevorzugt ist mindestens einer der mindestens zwei zapfenförmigen Erhebungen komplett in den Pelottengrundkörper eingebettet. Bevorzugt sind die mindestens zwei zapfenförmigen Erhebungen komplett in den Pelottengrundkörper eingebettet. Besonders bevorzugt ist das gesamte Pelottendruckelement, also die mindestens zwei zapfenförmigen Erhebungen und das Grundelement in den Pelottengrundkörper eingebettet, bevorzugt ist also das Pelottendruckelement vom Pelottengrundkörper ganz umschlossen, beispielsweise in den Pelottengrundkörper eingegossen.

Durch die bevorzugte vollständige Ummantelung des mindestens einen Pelottendruckelements mit dem Material des Pelottengrundkörpers ergibt sich nicht nur der beschriebene anwendungstechnische Vorteil, sondern auch in vorteilhafter Weise eine einfachere Herstellungsmöglichkeit der erfindungsgemäßen Pelotte, da somit die zweite Grundfläche des Pelottengrundkörpers, die dem Benutzer abgewandt ist, ohne weiteres plan herstellbar ist.

Bevorzugt ist eine Pelotte, umfassend einen Pelottengrundkörper aus einem Material mit einer ersten Härte und mindestens ein erfindungsgemäßes Pelottendruckelement aus einem Material mit einer zweiten Härte, wobei der Pelottengrundkörper eine erste, dem Benutzer zugewandte Grundfläche und eine zweite, dem Benutzer abgewandte Grundfläche aufweist, wobei die Oberfläche des mindestens einen Pelottendruckelements vollständig von dem Material des Pelottengrundkörpers umgeben ist.

Bevorzugt ist das Pelottendruckelement dabei ein Druckverstärkungselement. Ein Druckverstärkungselement dient einem punktuellen stärkeren Druck als der durch den Pelottengrundkörper ausgeübte Flächendruck.

In einer bevorzugten Ausführungsform weist der Pelottengrundkörper eine erste, dem Benutzer zugewandte Grundfläche und eine zweite, dem Benutzer abgewandte Grundfläche auf, wobei bevorzugt die zapfenförmigen Erhebungen von dem im Bereich der zweiten, dem Benutzer abgewandten Grundfläche positionierten Grundelement in Richtung der ersten, dem Benutzer zugewandten Grundfläche ragen.

In einer bevorzugten Ausführungsform ragen die mindestens zwei zapfenförmigen Erhebungen mindestens 2 mm in den Pelottengrundkörper hinein.

In einer bevorzugten Ausführungsform sind das Grundelement und die zapfenförmigen Erhebungen einstückig ausgebildet, insbesondere sind sie aus demselben Material gebildet.

In einer bevorzugten Ausführungsform ist das Grundelement des Pelottendruckelements stegförmig oder plattenförmig ausgebildet.

Erfindungsgemäß weist das Grundelement mindestens ein Loch auf, wobei die mindestens zwei zapfenförmigen Erhebungen auf dem Grundelement am Rand des mindestens einen Lochs positioniert sind.

Erfindungsgemäß sind die mindestens zwei zapfenförmigen Erhebungen über den das Loch überspannenden Steg miteinander verbunden.

Bevorzugt ist der Steg oder sind die Stege dem Grundelement zugeordnet beziehungsweise ist/sind Bestandteil von diesem.

In einer bevorzugten Ausführungsform sind am Rand des Lochs mindestens zwei Paare von zapfenförmigen Erhebungen positioniert, die jeweils über einen das Loch überspannenden Steg miteinander verbunden sind, wobei sich die Stege der beiden Paare von zapfenförmigen Erhebungen kreuzen, bevorzugt etwa in der Mitte des Lochs kreuzen.

Erfindungsgemäß befindet sich mindestens eine weitere zapfenförmige Erhebung auf mindestens einem Steg.

In einer bevorzugten Ausführungsform befindet sich eine weitere zapfenförmige Erhebung auf dem Kreuzungspunkt der beiden Stege.

Durch die erfindungsgemäße Ausgestaltung kann in vorteilhafter Weise gezielt ein spezifischer Druck durch die zapfenförmigen Erhebungen auf einen bestimmten Gewebepunkt ausgeübt werden. Ein solcher spezifischer Gewebepunkt kann insbesondere ein Triggerpunkt sein.

Wenn die Stege bevorzugt zusätzliche zapfenförmige Elemente aufweisen, beispielsweise am Kreuzungspunkt der Stege, führt das zu einem weiter verstärkten Massageeffekt, wenn diese auf den Stegen befindlichen zapfenförmigen Erhebungen eingedrückt werden und dadurch durch die Stege vermittelt die außen an den Stegen befindlichen zapfenförmigen Erhebungen bewegt werden, insbesondere sich von den auf den Stegen befindlichen zapfenförmigen Erhebungen wegbewegen oder sich zu diesen hinbewegen. Diese Bewegung führt zu weiteren Massageeffekten.

Die erfindungsgemäß vorgesehenen die zapfenförmigen Erhebungen verbindenden Stege können auch unterschiedlich breit und/oder unterschiedlich dick sein. Je nach Dickenverhältnis beziehungsweise Breitenverhältnis der Stege zueinander, kann die durch mehrere Stege verbundene, also aufgehängte zapfenförmige Erhebung gesteuert einfedern. Die zapfenförmige Erhebung neigt sich bei Einfederung in vorteilhafter Weise zu der Seite, an der der verbindende Steg im Vergleich zu den anderen Stegen schwächer und/oder dünner ausgeprägt ist.

In einer bevorzugten Ausführungsform ist der Abstand benachbarter zapfenförmiger Erhebungen mindestens 2,5-mal und höchstens 6-mal so weit wie die Höhe der zapfenförmigen Erhebungen. Besonders bevorzugt ist der Abstand benachbarter zapfenförmiger Erhebungen mindestens 3-mal und höchstens 5-mal so weit wie die Höhe der zapfenförmigen Erhebungen.

Beispielsweise sind etwa 3 mm hohe zapfenförmige Erhebungen bevorzugt etwa 7,5 mm bis 18 mm voneinander entfernt, bevorzugt 10 mm bis 14 mm, insbesondere etwa 12 mm.

In einer bevorzugten Ausführungsform haben die mindestens zwei zapfenförmigen Erhebungen die allgemeine Form eines Zapfens, beispielsweise einer Pyramide, eines Kegels, eines Zylinders, eines Hohlzylinders, eines Quaders, eines Hexaeders, eines Prismas, eines Polyeders, eines Stabes, einer Scheibe, eines Torus oder eines Rings. Die zapfenförmigen Erhebungen können auch stäbchenförmige Erhebungen sein.

Die zapfenförmigen Erhebungen haben ein erstes Ende, das am Grundelement liegt und den Zapfenfuß bildet, und ein zweites Ende, das den Zapfenkopf bildet. Der Zapfenkopf ist also der Bereich einer zapfenförmigen Erhebung, der am weitesten vom Grundelement entfernt liegt und der somit am weitesten seitlich verbogen werden kann.

Die zapfenförmigen Erhebungen zeigen vom Pelottengrundkörper in Richtung des Benutzers, der Zapfenkopf liegt also in Richtung der ersten Grundfläche des Pelottengrundkörpers und der Zapfenfuß und das Grundelement in Richtung der zweiten Grundfläche des Pelottengrundkörpers.

Dabei kann sich der Zapfenkopf bevorzugt um mindestens 1 mm seitlich verbiegen, besonders bevorzugt um mindestens 2 mm, besonders bevorzugt um mindestens 2,5 mm, wobei die Verbiegung bevorzugt in alle Richtungen erfolgen kann. Die Verbiegungsstrecke wird dabei vom Zapfenfuß aus gemessen, insbesondere vom Mittelpunkt des Zapfenfußes aus.

In einer bevorzugten Ausführungsform haben die mindestens zwei zapfenförmigen Erhebungen eine Höhe von mindestens 1 mm und höchsten 10 mm, beispielsweise von etwa 3 mm. In einer bevorzugten Ausführungsform haben die mindestens zwei zapfenförmigen Erhebungen eine Höhe von mindestens 2 mm. In einer bevorzugten Ausführungsform haben die mindestens zwei zapfenförmigen Erhebungen eine Höhe von mindestens 3 mm. Die Höhe bemisst sich von dem Grundelement aus.

Bevorzugt ist die Pelotte zumindest teilweise viskoelastisch, weist also bevorzugt mindestens einen viskoelastischen Teilbereich auf. Insbesondere ist die Pelotte bevorzugt zumindest im Bereich des Pelottendruckelements viskoelastisch.

Bevorzugt weist der Pelottengrundkörper zumindest im Bereich des Pelottendruckelements ein viskoelastisches Material auf. Bevorzugt ist der Pelottengrundkörper zumindest in einem Teilbereich viskoelastisch. Bevorzugt ist der Pelottengrundkörper viskoelastisch. Bevorzugt besteht der Pelottengrundkörper aus einem viskoelastischen Material.

Bevorzugt ist das Material des Pelottengrundkörpers Kunststoff, Silikon oder Gummi. Einem Fachmann sind aber auch weitere geeignete Materialien für einen Pelottengrundkörper aus dem Stand der Technik bekannt.

Beispielsweise kann es sich bei dem Material um thermoplastische Elastomere handeln. Geeignet sind beispielsweise auch Polyurethane.

Bevorzugt ist das Material des Pelottengrundkörpers biegbar und/oder dehnbar, so dass der Pelottengrundkörper beim Anlegen der Pelotte sich an die Körperform des Benutzers anpassen kann.

Bevorzugt ist das Pelottengrundkörpermaterial kein Textilmaterial. Der Pelottengrundkörper kann in einer alternativen Ausführungsform jedoch auch zusätzlich von einer textilen Ummantelung umgeben sein oder anderweitig beschichtet sein.

Das Pelottengrundkörpermaterial kann natürlich auch aus Mischungen von Materialen, insbesondere von mindestens zwei der genannten Materialen bestehen.

Bevorzugt ist das Material des Pelottendruckelements ein biegsames Material, insbesondere ein biegsamer Kunststoff. In einer bevorzugten Ausführungsform ist das Material des mindestens einen Pelottendruckelements Kunststoff, Silikon oder Gummi. Das Material des mindestens einen Pelottendruckelements kann natürlich auch aus Mischungen von Materialen, insbesondere von mindestens zwei der genannten Materialen bestehen.

Somit kann die erfindungsgemäße Zapfen-/Grundelementkonstruktion in vorteilhafter Weise einfach beispielsweise mittels Spritzgusstechnik hergestellt werden.

Erfindungsgemäß ist das erste Material des Pelottengrundkörpers weicher als das zweite Material des Pelottendruckelements.

In einer bevorzugten Ausführungsform hat das Material des Pelottengrundkörpers eine Shorehärte von mindestens 10 Shore OO und höchstens 50 Shore OO.

Bevorzugt hat das Material des Pelottengrundkörpers, also das erste Material eine Shorehärte von höchstens 45 Shore OO, bevorzugt höchsten 40 Shore OO, besonders bevorzugt höchsten 30 Shore OO. Bevorzugt hat das Material des Pelottengrundkörpers, also das erste Material eine Shorehärte von höchstens 25 Shore OO, bevorzugt höchsten 20 Shore OO, besonders bevorzugt höchsten 19 Shore OO. Bevorzugt hat das Material des Pelottengrundkörpers eine Shorehärte von mindestens 10 Shore OO, insbesondere mindestens 14 Shore OO, besonders bevorzugt von mindestens 15 Shore OO.

In einer bevorzugten Ausführungsform hat das Material des Pelottendruckelements eine Shorehärte von mindestens 10 Shore A, bevorzugt mindestens 14 Shore A und höchstens 80 Shore A. In einer bevorzugten Ausführungsform hat das Material des Pelottendruckelements hat eine Shorehärte von mindestens 20 Shore A und höchstens 80 Shore A.

In einer bevorzugten Ausführungsform weist das Material des mindestens einen Pelottendruckelements eine Shorehärte von mindestens 20 Shore A und höchstens 60 Shore A auf.

In einer bevorzugten Ausführungsform weist das Material des mindestens einen Pelottendruckelements eine Shorehärte von mindestens 20 Shore A, mehr bevorzugt von mindestens 25 Shore A auf.

In einer bevorzugten Ausführungsform weist das Material des mindestens einen Pelottendruckelements eine Shorehärte von höchstens 50 Shore A, auf. In einer bevorzugten Ausführungsform weist das Material des mindestens einen Pelottendruckelements eine Shorehärte von höchstens 49 Shore A, mehr bevorzugt von höchstens 45 Shore A auf.

Bevorzugt weist das Material des mindestens einen Pelottendruckelements eine Shorehärte von mindestens 35 bis höchsten 45 Shore A auf. Bevorzugt weist das Material des mindestens einen Pelottendruckelements eine Shorehärte von etwa 40 Shore A auf.

In einer bevorzugten Ausführungsform hat das Material des Pelottengrundkörpers eine Shorehärte von mindestens 10 Shore OO und höchstens 50 Shore OO und/oder das Material des Pelottendruckelements hat eine Shorehärte von mindestens 10 Shore A, bevorzugt mindestens 14 Shore A und höchstens 80 Shore A.

In einer bevorzugten Ausführungsform umfasst die Pelotte mindestens zwei biegsame Pelottendruckelemente, wobei die beiden Pelottendruckelemente über ein Verbindungselement miteinander verbunden sind. Besonders bevorzugt umfasst die Pelotte, insbesondere Kniepelotte, vier biegsame Pelottendruckelemente, wobei die vier Pelottendruckelemente über Verbindungselemente miteinander verbunden sind.

Die Verbindungselemente, die die Pelottendruckelemente mit mindestens zwei zapfenförmigen Erhebungen miteinander verbinden, können bevorzugt stegförmig oder plattenförmig ausgebildet sein.

Bevorzugt sind dabei zwei Pelottendruckelemente, umfassend jeweils zwei Löcher in dem Grundelement, insbesondere ein erstes Loch, an dessen Rand mindestens zwei Paare von zapfenförmigen Erhebungen positioniert sind, die jeweils über einen das Loch überspannenden Steg miteinander verbunden sind, wobei sich die Stege der beiden Paare von zapfenförmige Erhebungen kreuzen, bevorzugt etwa in der Mitte des Lochs kreuzen, und ein zweites Loch, an dessen Rand mindestens ein Paar von zapfenförmigen Erhebungen positioniert ist, das über einen das Loch überspannenden ersten Steg miteinander verbunden ist, wobei ein zweiter Steg das Loch überspannt, wobei sich der erste und der zweite Steg kreuzen, bevorzugt etwa in der Mitte des Lochs kreuzen, wobei die zwei Pelottendruckelemente über ein Verbindungselement miteinander verbunden sind.

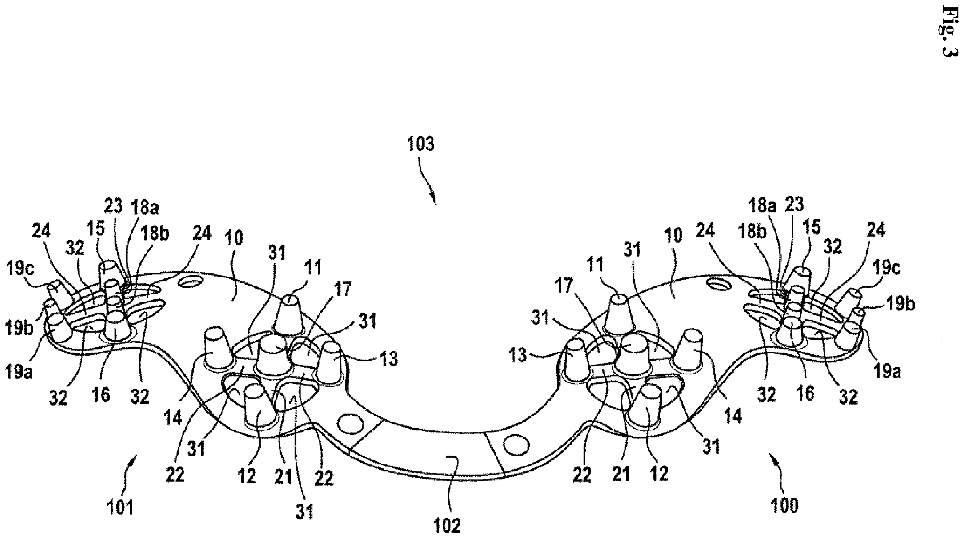

Bevorzugt sind zwei Pelottendruckelemente, umfassend jeweils ein biegsames Grundelement mit jeweils zwei Löchern mit zapfenförmigen Erhebungen und Stegen, wobei die Pelottendruckelemente über ein Verbindungselement miteinander verbunden sind, wie in Figur 3 gezeigt.

Bevorzugt sind die zwei oder mehr Pelottendruckelemente und das Verbindungselement einstückig und/oder aus demselben Material gefertigt.

Die Pelotte, insbesondere der durch die erste Grundoberfläche und zweite Grundoberfläche gebildete Teil des Pelottengrundkörpers kann jegliche Form, insbesondere jegliche dem Fachmann bekannte Pelottenform haben.

Beispielsweise kann die Pelotte ringförmig sein, insbesondere wenn sie als Patellapelotte verwendet wird. Alternativ kann die Form auch eine flache durchgehende Fläche sein. An einer oder mehrerer Seiten der Pelotten können flügelförmige Anhänge vorgesehen sein.

Bevorzugt ist die Pelotte eine Kniepelotte, eine Sprunggelenkspelotte, eine Rückenpelotte, eine Ellenbogenpelotte, eine Pelotte für den Armbereich oder eine Pelotte für den Bauchbereich.

In einer bevorzugten Ausführungsform weist die Pelotte mindestens zwei Pelottendruckelemente auf. In einer bevorzugten Ausführungsform weist die Pelotte mindestens zwei bis höchstens zwanzig Pelottendruckelemente auf. In einer bevorzugten Ausführungsform weist die Pelotte eine Vielzahl von Pelottendruckelementen auf.

Die Vielzahl von Pelottendruckelementen kann auf der ersten Grundfläche der Pelotte so positioniert sein, dass sie bei Verwendung der Pelotte den punktgenauen Druck auf spezifische Punkte, zum Beispiel auf Triggerpunkte, auf Akkupunkturpunkte oder auf infrapatellare Fettkörper, ausüben.

Die mindestens zwei Pelottendruckelemente, insbesondere eine Vielzahl von Pelottendruckelementen können in einer alternativen Ausführungsform auch über Stege im Pelottengrundkörper miteinander verbunden sein.

Neben dem mindestens einen Pelottendruckelement kann die Pelotte auch noch zusätzlich Friktionselemente aufweisen, beispielsweise aus dem Pelottengrundkörpermaterial.

In einer bevorzugten Ausführungsform ist die Pelotte eine Kniepelotte, eine Sprunggelenkspelotte, eine Rückenpelotte, eine Ellenbogenpelotte, eine Pelotte für den Armbereich oder eine Pelotte für den Bauchbereich. In einer bevorzugten Ausführungsform ist die Pelotte eine Kniepelotte, eine Sprunggelenkspelotte, eine Ellenbogenpelotte, eine Pelotte für den Armbereich oder eine Pelotte für den Bauchbereich. In einer bevorzugten Ausführungsform ist die Pelotte eine Kniepelotte, eine Sprunggelenkspelotte, eine Ellenbogenpelotte oder eine Pelotte für den Armbereich. In einer besonders bevorzugten Ausführungsform ist die Pelotte eine Kniepelotte.

In einer bevorzugten Ausführungsform ist die Pelotte eine Kniepelotte und weist mindestens zwei Pelottendruckelemente auf, wobei die zwei Pelottendruckelemente der Pelotte im Pelottengrundkörper positioniert sind, dass sie im angelegten Zustand der Pelotte im Bereich der infrapatellaren Fettkörper lokalisiert sind und auf diese Druck ausüben können.

Bevorzugt ist eine Pelotte, wobei das mindestens eine Pelottendruckelement der Pelotte im Pelottengrundkörper so positioniert sind, dass es im angelegten Zustand der Pelotte im Bereich eines Triggerpunkts lokalisiert ist und auf den Triggerpunkt Druck ausüben kann.

Bevorzugt ist eine erfindungsgemäße Pelotte zur Verwendung bei der Triggerpunkttherapie, wobei bevorzugt das mindestens eine Pelottendruckelement der Pelotte im Pelottengrundkörper so positioniert ist, dass es beim Tragen der Pelotte auf einen Triggerpunkt drückt.

Die Triggerpunkttherapie hat als Ziel die Beseitigung sogenannter myofaszialer Triggerpunkte. Diese sind lokal begrenzte Verhärtungen in der Skelettmuskulatur.

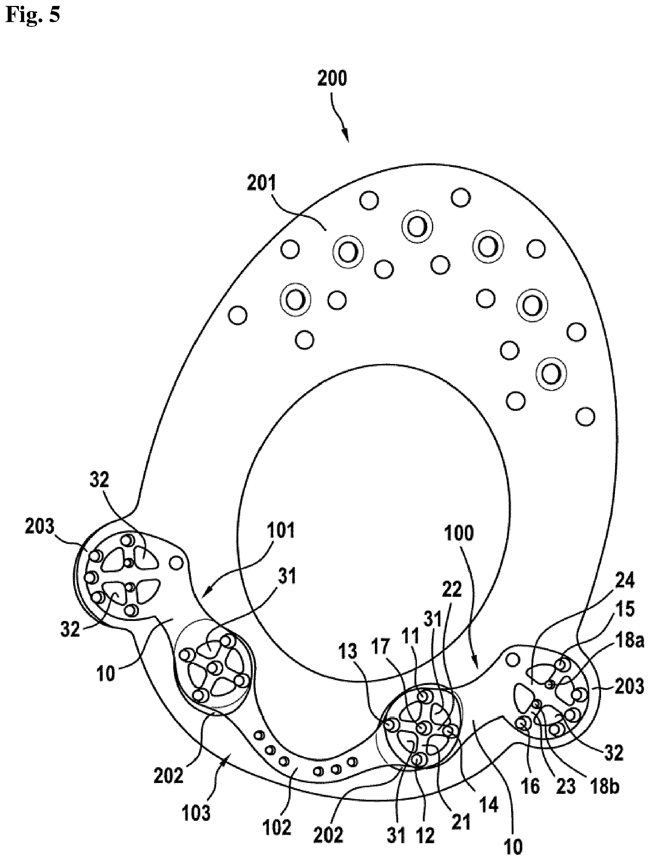

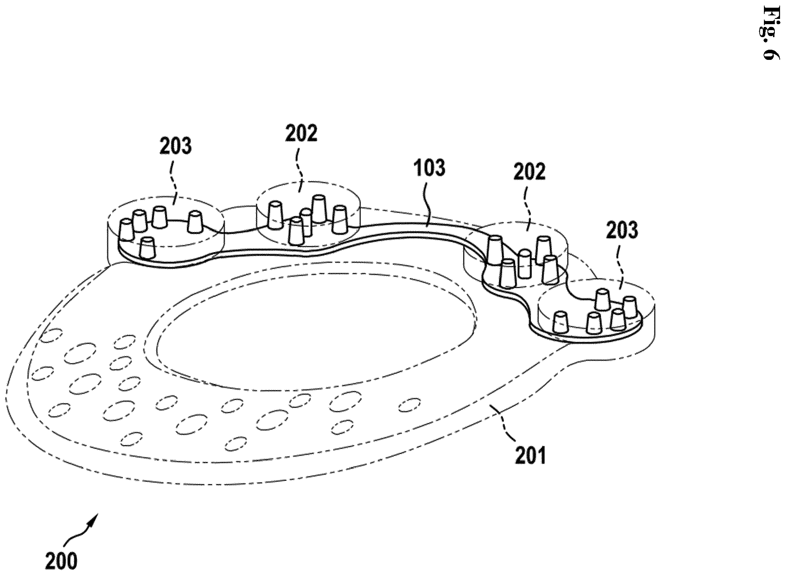

Bevorzugt ist eine Pelotte, insbesondere Kniepelotte, wie in den Figuren 5 und 6 gezeigt.

Die erfindungsgemäße Pelotte ist bevorzugt keine Pelotte für den Rücken. Die erfindungsgemäße Pelotte ist bevorzugt keine Rückenpelotte.

Die erfindungsgemäße Pelotte ist bevorzugt keine Schuheinlage oder Fußeinlage.

Die Pelotte ist bevorzugt für ein orthopädisches Hilfsmittel. Die Pelotte ist bevorzugt Bestandteil eines orthopädischen Hilfsmittels. Das orthopädische Hilfsmittel kann eine Orthese oder eine Bandage sein.

Die vorliegende Erfindung betrifft auch ein orthopädisches Hilfsmittel umfassend eine erfindungsgemäße Pelotte. Die vorliegende Erfindung betrifft auch eine Orthese oder eine Bandage umfassend eine erfindungsgemäße Pelotte.

In einer bevorzugten Ausführungsform ist die Orthese oder Bandage eine Knieorthese oder Kniebandage, eine Sprunggelenksorthese oder -bandage, eine Rückenorthese oder -bandage, eine Ellenbogenorthese oder -bandage, eine Orthese oder Bandage für den Armbereich oder eine Orthese oder Bandage für den Bauchbereich. In einer bevorzugten Ausführungsform ist die Orthese oder Bandage eine Knieorthese oder Kniebandage, eine Sprunggelenksorthese oder -bandage, eine Ellenbogenorthese oder -bandage, eine Orthese oder Bandage für den Armbereich oder eine Orthese oder Bandage für den Bauchbereich. In einer bevorzugten Ausführungsform ist die Orthese oder Bandage eine Knieorthese oder Kniebandage, eine Sprunggelenksorthese oder -bandage, eine Ellenbogenorthese oder -bandage, oder eine Orthese oder Bandage für den Armbereich.

In einer bevorzugten Ausführungsform ist die Orthese oder Bandage eine Knieorthese oder Kniebandage.

Bevorzugt ist die Orthese eine elastische Gestrickorthese oder die Bandage eine elastische Gestrickbandage, insbesondere eine Kniebandage.

In einer bevorzugten Ausführungsform ist die Orthese oder Bandage keine Rückenorthese oder Rückenbandage.

Die vorliegende Erfindung betrifft auch die Verwendung einer erfindungsgemäßen Pelotte als Druckpelotte, bevorzugt in der Triggerpunkttherapie.

Die vorliegende Erfindung betrifft auch ein Verfahren zur Triggerpunkttherapie, bei dem eine erfindungsgemäße Pelotte an einem Benutzer in einem ersten Schritt so angelegt wird, dass das mindestens eine Pelottendruckelement auf einen Triggerpunkt drückt und in einem zweiten Schritt die erfindungsgemäße Pelotte in dieser Position von dem Benutzer über einen bestimmten Zeitraum getragen wird.

Weitere bevorzugte Gegenstände der Erfindung ergeben sich aus den Unteransprüchen und den Nebenansprüchen.

Die Erfindung wird anhand der nachfolgenden Figuren näher beschrieben, ohne dass die dort dargestellten Ausführungsformen der Erfindung beschränkend zu verstehen sind.
Figur 1 zeigt den Grundaufbau einer erfindungsgemäßen Pelotte.
Figur 2 zeigt eine bevorzugte Ausführungsform eines Pelottendruckelements einer erfindungsgemäßen Pelotte.
Figur 3 zeigt eine bevorzugte Ausführungsform von zwei miteinander verbundenen Pelottendruckelementen einer erfindungsgemäßen Pelotte.
Figur 4 zeigt eine Seitenansicht der zwei miteinander verbundenen Pelottendruckelemente aus Figur 3.
Figur 5 zeigt eine erfindungsgemäße Pelotte mit den Pelottendruckelementen aus Figur 3.
Figur 6 zeigt die erfindungsgemäße Pelotte aus Figur 5 in Schrägansicht.
Figur 7 zeigt die Innenseite einer gestrickten Kniebandage mit einer erfindungsgemäßen Pelotte.

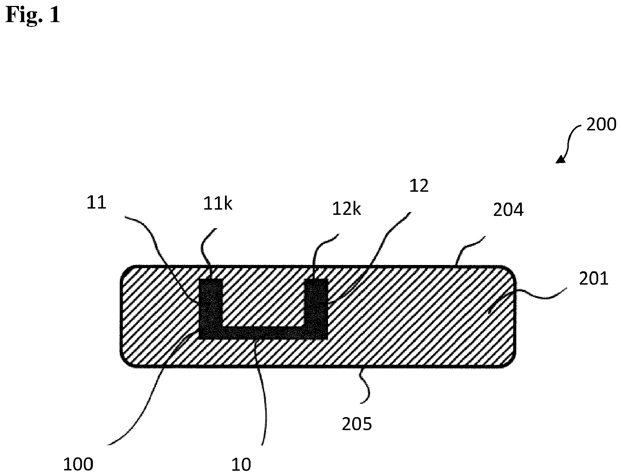

Figur 1 zeigt eine erfindungsgemäße Pelotte (200), die einen Pelottengrundkörper (201) aufweist, in den ein Pelottendruckelement (100) eingebettet ist. Das Pelottendruckelement (100) ist einstückig ausgestaltet und besteht aus einem biegsamen Material, das härter ist als das Material des Pelottengrundkörpers (201). Die Pelotte (200) hat eine erste Seite mit einer ersten Grundfläche (204) und eine gegenüberliegende zweite Seite mit einer zweiten Grundfläche (205). Bei Verwendung der Pelotte (200) ist die erste Grundfläche (204) in Richtung des Benutzers gerichtet, wird also auf die Haut des Benutzers gedrückt, wobei natürlich zwischen Pelotte (200) und Haut noch eine Zwischenschicht liegen kann, beispielsweise eine Kaschierungsschicht aus Stoff.

Das Pelottendruckelement (100) weist eine erste zapfenförmige Erhebung (11) und eine zweite zapfenförmige Erhebung (12) auf, wobei die beiden zapfenförmigen Erhebungen (11,12) an ihren Zapfenfüßen über ein biegsames Grundelement (10) miteinander verbunden sind. Das Grundelement (10) ist im Pelottengrundkörper (201) in Richtung der zweiten Grundfläche (205) positioniert, während die zapfenförmigen Erhebungen in Richtung der ersten Grundfläche (204) verlaufen, in deren Nähe somit die Zapfenköpfe (11k, 12k) liegen.

Wird nun die Pelotte (200), beispielsweise mittels einer Bandage, auf die Haut des Benutzers gedrückt, so bewegen sich die zapfenförmigen Erhebungen (11,12), insbesondere im Bereich ihrer Köpfe (11k, 12k) aufeinander zu oder voneinander weg. Somit resultiert der senkrechte Druck in einer seitlichen Bewegung der zapfenförmigen Erhebungen (11,12). Diese Seitwärtsbewegung der zapfenförmigen Erhebungen (11,12) führt zu einem verbesserten Massageeffekt, der über einen einfachen senkrechten Druck der Zapfenköpfe (1 1k, 12k) auf die Haut des Benutzers deutlich hinausgeht. Durch die verschiedenen Druckeinflüsse bei der Bewegung des Nutzers ergibt sich eine chaotische Seitwärtsbewegung der zapfenförmigen Erhebungen (11,12), die die chaotische Bewegung bei einer manuellen Massage imitiert.

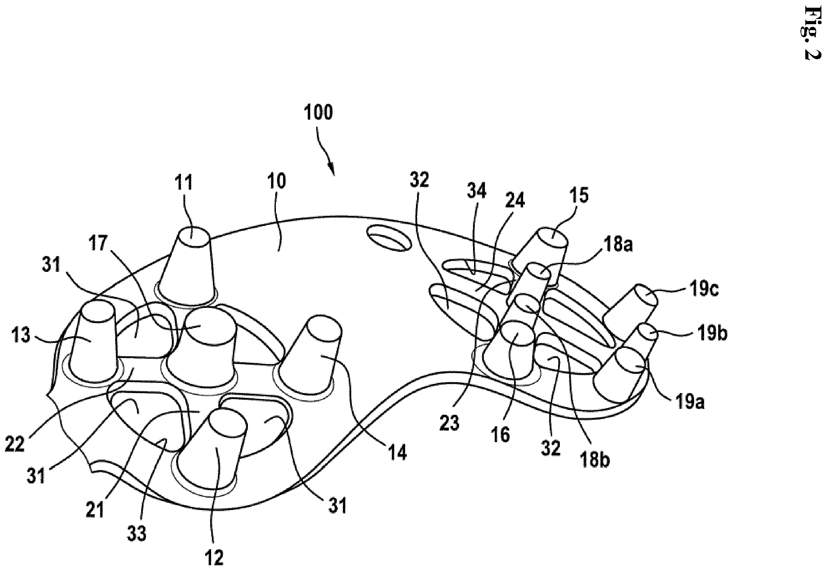

Figur 2 zeigt eine bevorzugte Ausführungsform eines Pelottendruckelements (100) einer erfindungsgemäßen Pelotte, hier einer Kniepelotte. Das Pelottendruckelement (100) weist ein biegsames Grundelement (10) mit zwei Löchern (31,32) auf. Bei dem ersten Loch (31) sind am Lochrand (33) vier zapfenförmige Erhebungen (11,12,13,14) angeordnet. Dabei bilden die vier zapfenförmigen Erhebungen ein erstes gegenüberliegendes Paar (11,12) und ein zweites gegenüberliegendes Paar (13,14). Das erste Paar an zapfenförmigen Erhebungen (11,12) ist über einen ersten Steg (21) miteinander verbunden. Auch das zweite Paar an zapfenförmigen Erhebungen (13,14) ist über einen zweiten Steg (22) miteinander verbunden. Die beiden Stege (21,22) kreuzen sich in der Mitte des Lochs (31). Im Kreuzungspunkt der Stege (21,22) befindet sich eine weitere mittlere zapfenförmige Erhebung (17). Der biegsame Grundflächenkörper (10) weist ein zweites Loch (32) auf, am Lochrand (34) des zweiten Lochs (32) befinden sich zwei gegenüberliegende zapfenförmige Erhebungen (15,16), die ebenfalls über einen Steg (23), der das Loch (32) überspannt, miteinander verbunden sind. Dieser Steg (23) weist zwei weitere, mittlere zapfenförmige Erhebungen (18a, 18b) auf. Der erste Steg (23) wird von einem zweiten, ebenfalls das Loch (32) überspannenden Steg (24) gekreuzt, der im Bereich von drei weiteren am Lochrand (34) liegenden stäbchenförmigen Erhebungen (19a,19b,19c) am Grundelement (10) ansetzt.

Das Pelottendruckelement (100) ist aus einem einzigen biegsamen Kunststoff im Spritzgussverfahren gegossen worden.

Figur 3 zeigt eine bevorzugte Ausführungsform von zwei Pelottendruckelementen (100,101) einer erfindungsgemäßen Pelotte, die über ein Verbindungselement (102) miteinander verbunden sind und so eine Einheit (103) bilden. Das Pelottendruckelement (100) entspricht dem Pelottendruckelement aus Figur 2. Zu sehen sind wieder die zapfenförmigen Erhebungen (11,12,13,14,15,16,17,18a,18b,19a,19b,19c), die sich im Bereich der zwei Löcher (31,32) befinden, sowie die die Löcher überspannenden Stege (21,22,23,24). Das Grundelement (10) ist über ein Verbindungselement (102) mit dem Grundelement (10) des zweiten Pelottendruckelements (101) verbunden. Das zweite Pelottendruckelement (101) ist spiegelverkehrt zu dem ersten Pelottendruckelement (100), zeigt aber wiederum die zapfenförmigen Erhebungen (11,12,13,14,15,16,17,18a,18b,19a,19b,19c), die im Bereich der beiden Löcher (31,32) positioniert sind, wobei die Löcher von den Stegen (21,22,24) überspannt werden. Eine solche Einheit (103) von zwei Pelottendruckelementen (100,101) eignet sich insbesondere beim Einsatz in einer Kniepelotte einer Kniebandage.

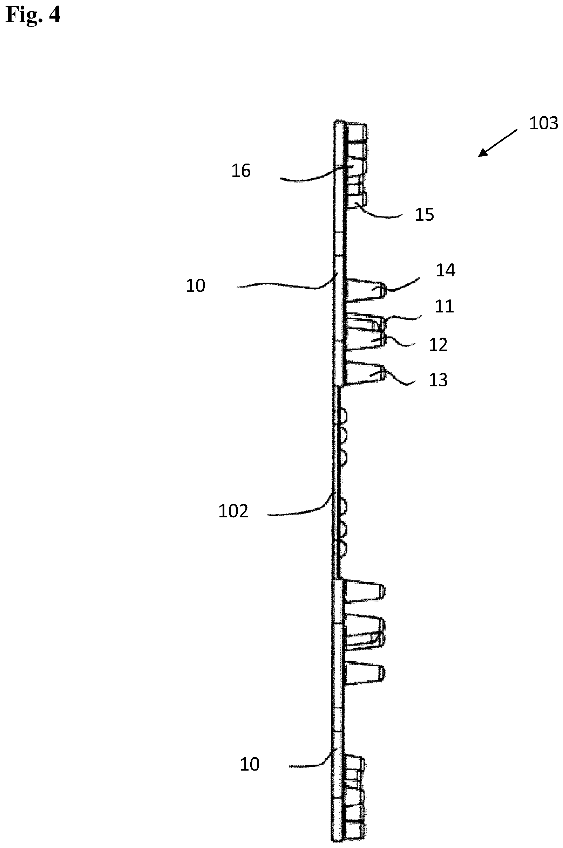

Figur 4 zeigt die Pelottendruckelementeinheit (103) aus Figur 3 in Seitenansicht. Zu sehen sind die beiden Grundelemente (10), die über das Verbindungselement (102) miteinander verbunden sind. Aus den Grundelementen (10) ragen die in Figur 3 gezeigten zapfenförmigen Erhebungen (11,12,13,14,15,16) heraus.

Figur 5 zeigt eine Kniepelotte (200) mit einem ringförmigen Pelottengrundkörper (201). Der Pelottengrundkörper (201) weist zwei noppenförmige Vorsprünge (202) und zwei flügelförmige Vorsprünge (203) auf, wie sie beispielsweise aus der DE 10 2011 010 827 A1 bekannt sind und die dort beschriebene Funktion erfüllen, also zum einen im Bereich der infrapatellaren Fettkörper lokalisiert sind und auf diese Druck ausüben können beziehungsweise mit der infrapatellaren Gelenkspalte in Eingriff kommen. Gezeigt ist dabei die Draufsicht auf die Pelotte (200), so dass die erste, dem Benutzer zugewandte Oberfläche der Pelotte zu sehen ist. Die Einheit (103) aus Figur 3 aus den zwei Pelottendruckelementen (100,101), die über ein Verbindungselement (102) miteinander verbunden sind, ist in den Pelottengrundkörper (201) komplett eingebettet und zwar derart, dass das biegsame Grundelement (10) jeweils so positioniert ist, dass die ersten Löcher (31) mit den zugehörigen zapfenförmigen Erhebungen (11,12,13,14,17) und Stegen (21,22) in den noppenförmigen Vorsprüngen (202) liegen und die zweiten Löcher (32) mit den zugehörigen zapfenförmigen Erhebungen (15,16,18a,18b) und den Stegen (23,24) im Bereich der flügelförmigen Vorsprünge (203) positioniert sind.

Somit können die durch die erfindungsgemäße Ausgestaltung hervorgerufenen Massageeffekte an den wichtigen Bereichen unterhalb und neben der Kniescheibe wirken.

Figur 6 zeigt die Kniepelotte (200) aus Figur 5 in Schrägansicht. Die Einheit (103) aus den zwei Pelottendruckelementen und dem Verbindungselement ist einstückig und komplett in den Pelottengrundkörper (201) eingegossen. Dabei liegen die verschiedenen zapfenförmigen Erhebungen im Bereich der noppenförmigen Vorsprünge (202) und der flügelförmigen Vorsprünge (203) des Pelottengrundkörpers (201). Dadurch wird in vorteilhafter Weise sowohl eine Druck- als auch eine Massageausübung im Bereich der infrapatellaren Fettkörper ausgeübt.

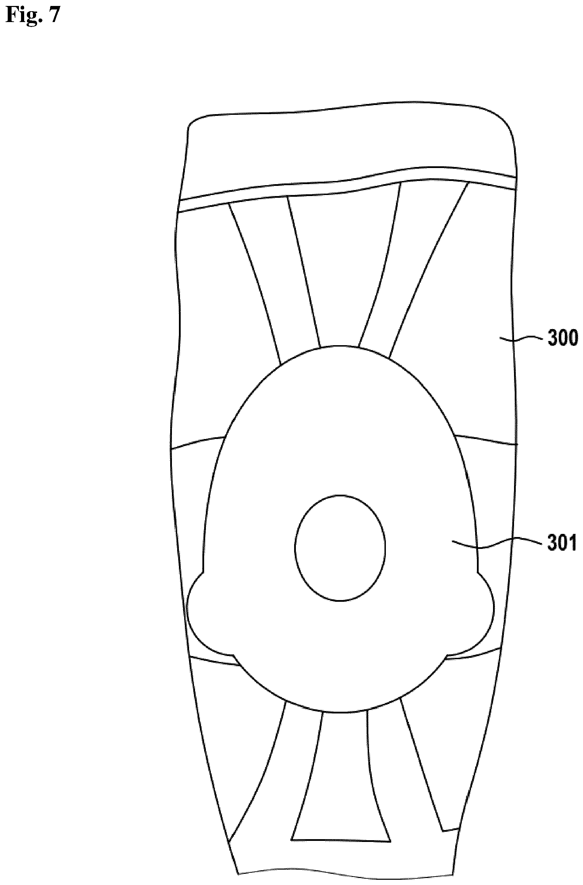

Figur 7 zeigt eine erfindungsgemäße Kniebandage (300) aus einem Gestrick, die eine erfindungsgemäße Pelotte aufweist. Zu sehen ist dabei die Innenseite der Bandage (300). Die Pelotte liegt auf der Innenseite des Bandagegestricks auf und ist dort über ein Kaschiermaterial (301) positioniert und befestigt, so dass in der Figur nur die Kontur der Pelotte zu erkennen ist. Beim Tragen der Kniebandage (300) liegt die Pelotte im Bereich des Knies und umschließt die Kniescheibe, sodass die flügelförmigen Vorsprünge und noppenförmige Vorsprünge im Bereich der infrapatellaren Fettkörper zu liegenkommen und die Pelotte mit den zapfenförmigen Erhebungen dort wirken kann.

### Bezugszeichenliste:

- 10: Grundelement
- 11: zapfenförmige Erhebung
- 11k: Zapfenkopf
- 12: zapfenförmige Erhebung
- 12k: Zapfenkopf
- 13: zapfenförmige Erhebung
- 14: zapfenförmige Erhebung
- 15: zapfenförmige Erhebung
- 16: zapfenförmige Erhebung
- 17: zapfenförmige Erhebung
- 18a: zapfenförmige Erhebung
- 18b: zapfenförmige Erhebung
- 19a: zapfenförmige Erhebung
- 19b: zapfenförmige Erhebung
- 19c: zapfenförmige Erhebung
- 21: erster Steg
- 22: zweiter Steg
- 23: Steg
- 24: Steg
- 31: Loch
- 32: Loch
- 33: Lochrand
- 34: Lochrand
- 100: Pelottendruckelement
- 101: Pelottendruckelement
- 102: Verbindungselement
- 103: zwei miteinander über ein Verbindungselement verbundene Pelottendruckelemente
- 200: Pelotte
- 201: Pelottengrundkörper
- 202: noppenförmige Vorsprünge
- 203: flügelförmige Vorsprünge
- 204: erste Grundfläche
- 205: zweite Grundfläche
- 300: Strickbandage
- 301: Kaschiermaterial

## Patentansprüche

1. Pelotte (200) für orthopädische Hilfsmittel, umfassend einen Pelottengrundkörper (201) aus einem ersten Material und mindestens einem Pelottendruckelement (100,101) aus einem zweiten Material, wobei das Pelottendruckelement (100,101) zumindest teilweise in den Pelottengrundkörper (201) eingebettet ist und wobei das erste Material des Pelottengrundkörpers (201) weicher ist als das zweite Material des Pelottendruckelements (100,101), wobei das Pelottendruckelement (100,101) mindestens zwei zapfenförmige Erhebungen (11,12,13,14) und ein Grundelement (10) aufweist, wobei die zapfenförmigen Erhebungen (11,12,13,14) auf dem Grundelement (10) angeordnet sind, wobei die zapfenförmigen Erhebungen (11,12,13,14) über das Grundelement (10) viskoelastisch beweglich sind, wobei das Grundelement (10) mindestens ein Loch (31,32) aufweist und wobei die mindestens zwei zapfenförmige Erhebungen (11,12,13,14) auf dem Grundelement (10) am Rand (33,34) des mindestens einen Lochs (31,32) positioniert sind, **dadurch gekennzeichnet dass** die mindestens zwei zapfenförmigen Erhebungen (11,12,13,14) über einen das Loch (31,32) überspannenden Steg (21,22,23,24) miteinander verbunden sind und sich mindestens eine weitere zapfenförmige Erhebung (17, 18a, 18b) auf dem Steg (21,22,23,24) befindet.

2. Pelotte nach Anspruch 1, **dadurch gekennzeichnet, dass** zumindest ein Teilbereich der mindestens zwei zapfenförmigen Erhebungen (11,12,13,14) in den Pelottengrundkörper (10) eingebettet ist.

3. Pelotte nach einem der vorhergehenden Ansprüche, **dadurch gekennzeichnet, dass** das Grundelement (10) des Pelottendruckelements (100,101) stegförmig oder plattenförmig ausgebildet ist.

4. Pelotte nach einem der vorhergehenden Ansprüche, **dadurch gekennzeichnet, dass** die Pelotte mindestens zwei biegsame Pelottendruckelemente (100,101) umfasst, wobei die beiden Pelottendruckelemente (100,101) über ein Verbindungselement (102) miteinander verbunden sind.

5. Pelotte nach einem der vorhergehenden Ansprüche, **dadurch gekennzeichnet, dass** die mindestens zwei zapfenförmigen Erhebungen (11,12,13,14) mindestens 2 mm in den Pelottengrundkörper (10) hineinragen.

6. Pelotte nach einem der vorhergehenden Ansprüche, **dadurch gekennzeichnet, dass** an am Rand (33,34) des Lochs (31,32) mindestens zwei Paare von zapfenförmigen Erhebungen (11,12,13,14) positioniert sind, die jeweils über einen das Loch (31,32) überspannenden Steg (21,22,23,24) miteinander verbunden sind, wobei sich die Stege (21,22,23,24) der beiden Paare von zapfenförmige Erhebungen (11,12,13,14) kreuzen, bevorzugt etwa in der Mitte des Lochs (32,32) kreuzen.

7. Pelotte nach Anspruch 6 **dadurch gekennzeichnet, dass** sich eine weitere zapfenförmige Erhebung (17,18a,18b) auf und/oder neben dem Kreuzungspunkt der beiden Stege (21,22,23,24) befindet.

8. Pelotte nach einem der vorhergehenden Ansprüche, **dadurch gekennzeichnet, dass** das Grundelement (10) und die zapfenförmigen Erhebungen (11,12,13,14) einstückig ausgebildet sind, insbesondere aus demselben Material gebildet sind.

9. Pelotte nach einem der vorhergehenden Ansprüche, **dadurch gekennzeichnet, dass** der Pelottengrundkörper (201) eine erste, dem Benutzer zugewandte Grundfläche (204)und eine zweite, dem Benutzer abgewandte Grundfläche (205) aufweist und wobei die zapfenförmigen Erhebungen (11,12,13,14) von dem im Bereich der zweiten, dem Benutzer abgewandten Grundfläche (205) positionierten Grundelements (10) in Richtung der ersten, dem Benutzer zugewandten Grundfläche (204) ragen.

10. Pelotte nach einem der vorhergehenden Ansprüche, **dadurch gekennzeichnet, dass** das Material des Pelottengrundkörpers (201) eine Shorehärte von mindestens 10 Shore-OO und höchstens 50 Shore-OO hat und/oder das Material des Pelottendruckelements (100,101) eine Shorehärte von mindestens 10 Shore-A und höchstens 80 Shore-A hat.

11. Pelotte nach einem der vorhergehenden Ansprüche, **dadurch gekennzeichnet, dass** der Abstand benachbarter zapfenförmiger Erhebungen (11,12,13,14) mindestens 2,5-mal und höchstens 6-mal so weit ist wie die Höhe der zapfenförmigen Erhebungen (11,12,13,14).

12. Pelotte nach einem der vorhergehenden Ansprüche, **dadurch gekennzeichnet, dass** die Pelotte eine Kniepelotte, eine Sprunggelenkspelotte, eine Rückenpelotte, eine Ellenbogenpelotte, eine Pelotte für den Armbereich oder eine Pelotte für den Bauchbereich ist.

13. Pelotte nach einem der vorhergehenden Ansprüche, **dadurch gekennzeichnet, dass** die Pelotte eine Kniepelotte ist und mindestens zwei Pelottendruckelemente (100,101) aufweist, wobei die zwei Pelottendruckelemente (100,101) der Pelotte im Pelottengrundkörper (201) positioniert sind, dass sie im angelegten Zustand der Pelotte im Bereich der infrapatellaren Fettkörper lokalisiert sind und auf diese Druck ausüben können.

14. Orthese oder Bandage (300) umfassend eine Pelotte (200) nach einem der vorhergehenden Ansprüche.

## Claims

1. A pad (200) for orthopedic aids, comprising a pad base body (201) made of a first material and at least one pad pressure element (100, 101) made of a second material, wherein the pad pressure element (100, 101) is at least partially embedded in the pad base body (201), and wherein the first material of the pad base body (201) is softer than the second material of the pad pressure element (100, 101), wherein the pad pressure element (100, 101) has at least two pin-shaped elevations (11, 12, 13, 14) and a base element (10), wherein the pin-shaped elevations (11, 12, 13, 14) are arranged on the base element (10), wherein the pin-shaped elevations (11, 12, 13, 14) are movable in a viscoelastic manner by way of the base element (10), wherein the base element (10) has at least one hole (31, 32) and wherein the at least two pin-shaped elevations (11, 12, 13, 14) on the base element (10) are positioned at the edge (33, 34) of the at least one hole (31, 32) **characterized in that** the at least two pin-shaped elevations (11, 12, 13, 14) are connected to one another by way of a web (21, 22, 23, 24) spanning the hole (31, 32) and a further pin-shaped elevation (17, 18a, 18b) is arranged on the web (21, 22, 23, 24).

2. The pad according to claim 1, **characterized in that** at least a portion of the at least two pin-shaped elevations (11, 12, 13, 14) is embedded in the pad base body (10).

3. The pad according to one of the preceding claims, **characterized in that** the base element (10) of the pad pressure element (100, 101) is web-shaped or plate-shaped.

4. The pad according to one of the preceding claims, **characterized in that** the pad comprises at least two flexible pad pressure elements (100, 101), wherein the two pad pressure elements (100, 101) are connected to one another by way of a connecting element (102).

5. The pad according to one of the preceding claims, **characterized in that** the at least two pin-shaped elevations (11, 12, 13, 14) protrude at least 2 mm into the pad base body (10).

6. The pad according to one of the preceding claims, **characterized in that** at least two pairs of pin-shaped elevations (11, 12, 13, 14) are positioned at the edge (33, 34) of the hole (31, 32) and are in each case connected to one another by way of a web (21, 22, 23, 24) spanning the hole (31, 32), wherein the webs (21, 22, 23, 24) of the two pairs of pin-shaped elevations (11, 12, 13, 14) intersect, preferably approximately in the center of the hole (32, 32).

7. The pad according to claim 6, **characterized in that** a further pin-shaped elevation (17, 18a, 18b) is arranged on and/or next to the intersecting point of the two webs (21, 22, 23, 24).

8. The pad according to one of the preceding claims, **characterized in that** the base element (10) and the pin-shaped elevations (11, 12, 13, 14) are designed in one piece, in particular are made of the same material.

9. The pad according to one of the preceding claims, **characterized in that** the pad base body (201) has a first base surface (204) facing the user and a second base surface (205) facing away from the user and wherein the pin-shaped elevations (11, 12, 13, 14) project from the base element (10) positioned in the region of the second base surface (205) facing away from the user in the direction of the first base surface (204) facing the user.

10. The pad according to one of the preceding claims, **characterized in that** the material of the pad base body (201) has a Shore hardness of at least 10 Shore OO and at most 50 Shore OO and/or the material of the pad pressure element (100, 101) has a Shore hardness of at least 10 Shore A and at most 80 Shore A.

11. The pad according to one of the preceding claims, **characterized in that** the distance between adjacent pin-shaped elevations (11, 12, 13, 14) is at least 2.5 times and at most 6 times as wide as the height of the pin-shaped elevations (11, 12, 13, 14).

12. The pad according to one of the preceding claims, **characterized in that** the pad is a knee pad, an ankle pad, a back pad, an elbow pad, a pad for the arm region or a pad for the abdominal region.

13. The pad according to one of the preceding claims, **characterized in that** the pad is a knee pad and has at least two pad pressure elements (100, 101), wherein the two pad pressure elements (100, 101) of the pad are positioned in the pad base body (201) so that they are located in the region of the infrapatellar fat pads when the pad is applied and can exert pressure thereon.

14. An orthosis or a bandage (300) comprising a pad (200) according to one of the preceding claims.

## Revendications

1. Coussinet (200) pour aides orthopédiques, comprenant un corps de base de coussinet (201) réalisé dans un premier matériau et au moins un élément de pression de coussinet (100, 101) réalisé dans un second matériau, dans lequel l'élément de pression de coussinet (100, 101) est au moins partiellement intégré dans le corps de base de coussinet (201), et dans lequel le premier matériau du corps de base de coussinet (201) est plus souple que le second matériau de l'élément de pression de coussinet (100, 101), dans lequel l'élément de pression de coussinet (100, 101) a au moins deux élévations en forme de pin (11, 12, 13, 14) et un élément de base (10), dans lequel les élévations en forme de pin (11, 12, 13, 14) sont disposées sur l'élément de base (10), dans lequel les élévations en forme de pin (11, 12, 13, 14) sont mobiles de manière viscoélastique au moyen de l'élément de base (10), dans lequel l'élément de base (10) a au moins un trou (31, 32) et dans lequel les au moins deux élévations en forme de pin (11, 12, 13, 14) sur l'élément de base (10) sont positionnées au bord (33, 34) du au moins un trou (31, 32) **caractérisé en ce que** les au moins deux élévations en forme de pin (11, 12, 13, 14) sont connectées l'une à l'autre au moyen d'une barre (21, 22, 23, 24) enjambant le trou (31, 32) et qu'une autre élévation en forme de pin (17, 18a, 18b) est disposée sur la barre (21, 22, 23, 24).

2. Le coussinet selon la revendication 1, **caractérisé en ce qu'**au moins une partie des au moins deux élévations en forme de pin (11, 12, 13, 14) est intégrée dans le corps de base du coussinet (10).

3. Le coussinet selon l'une des revendications précédentes, **caractérisé en ce que** l'élément de base (10) de l'élément de pression de coussinet (100, 101) est en forme de barre ou de plaque.

4. Le coussinet selon l'une des revendications précédentes, **caractérisé en ce que** le coussinet comprend au moins deux éléments de pression de coussinet flexibles (100, 101), dans lequel les deux éléments de pression de coussinet (100, 101) sont connectés l'un à l'autre au moyen d'un élément de connexion (102).

5. Le coussinet selon l'une des revendications précédentes, **caractérisé en ce que** les au moins deux élévations en forme de pin (11, 12, 13, 14) dépassent d'au moins 2 mm dans le corps de base du coussinet (10).

6. Le coussinet selon l'une des revendications précédentes, **caractérisé en ce qu'**au moins deux paires d'élévations en forme de pin (11, 12, 13, 14) sont positionnées au bord (33, 34) du trou (31, 32) et sont dans chaque cas connectées l'une à l'autre au moyen d'une barre (21, 22, 23, 24) enjambant le trou (31, 32), dans lequel les barres (21, 22, 23, 24) des deux paires d'élévations en forme de pin (11, 12, 13, 14) se croisent, préférablement approximativement au centre du trou (32, 32).

7. Le coussinet selon la revendication 6, **caractérisé en ce qu'**une autre élévation en forme de pin (17, 18a, 18b) est disposée sur et/ou à côté du point de croisement des deux barres (21, 22, 23, 24).

8. Le coussinet selon l'une des revendications précédentes, **caractérisé en ce que** l'élément de base (10) et les élévations en forme de pin (11, 12, 13, 14) sont conçus d'une seule pièce, en particulier sont réalisés dans le même matériau.

9. Le coussinet selon l'une des revendications précédentes, **caractérisé en ce que** le corps de base de coussinet (201) a une première surface de base (204) orientée vers l'utilisateur et une deuxième surface de base (205) orientée à l'opposé de l'utilisateur et dans lequel les élévations en forme de pin (11, 12, 13, 14) font saillie de l'élément de base (10) positionné dans la région de la deuxième surface de base (205) orientée à l'opposé de l'utilisateur dans la direction de la première surface de base (204) orientée vers l'utilisateur.

10. Le coussinet selon l'une des revendications précédentes, **caractérisé en ce que** le matériau du corps de base de coussinet (201) a une dureté Shore d'au moins 10 Shore OO et d'au plus 50 Shore OO et/ou le matériau de l'élément de pression de coussinet (100, 101) a une dureté Shore d'au moins 10 Shore A et d'au plus 80 Shore A.

11. Le coussinet selon l'une des revendications précédentes, **caractérisé en ce que** la distance entre les élévations en forme de pin adjacentes (11, 12, 13, 14) est au moins 2,5 fois et au plus 6 fois plus large que la hauteur des élévations en forme de pin (11, 12, 13, 14).

12. Le coussinet selon l'une des revendications précédentes, **caractérisé en ce que** le coussinet est une genouillère, une chevillère, une dorsale, une coudière, un coussinet pour la région du bras ou un coussinet pour la région abdominale.

13. Le coussinet selon l'une des revendications précédentes, **caractérisé en ce que** le coussinet est une genouillère et a au moins deux éléments de pression de coussinet (100, 101), dans lequel les deux éléments de pression de coussinet (100, 101) du coussinet sont positionnés dans le corps de base de coussinet (201) de manière à être localisés dans la région des coussinets de graisse infrapatellaires lorsque le coussinet est appliqué et à pouvoir exercer une pression sur ces coussinets.

14. Orthèse ou bandage (300) comprenant un coussinet (200) selon l'une des revendications précédentes.
